# EUROPEAN PATENT APPLICATION

(11) **EP 1 382 662 A1**
(43) Date of publication of application: **21.01.2004**
(21) Application number: 02722766.9
(22) Date of filing: 24.04.2002
(51) Int. Cl.: C11C 3/00, A61K 31/575, A61P 3/06, C07J 9/00, A23D 9/00, A23L 1/30

(54) **STEROL COMPOSITIONS, FAT COMPOSITIONS CONTAINING THE SAME AND FOODS**

(30) Priority: 27.04.2001 JP 2001132518
(71) Applicant: Ajinomoto Co., Inc., Tokyo 104-8315 (JP); Ajinomoto Oil Mills Co., Ltd., Yokohama-shi, Kanagawa 230-0053 (JP)
(72) Inventor: TASHIMA, I., c/o Ajinomoto Oil Mills Co. Inc., Yokohama-shi, Kanagawa 230-0053 (JP); ISHIZAKI, T., c/o Ajinomoto Co. Inc., Kawasaki-shi, Kanagawa 210-8681 (JP); MORI, O., c/o Ajinomoto Oil Mills Co. Inc., Yokohama-shi, Kanagawa 230-0053 (JP); BABA, E., c/o Ajinomoto Oil Mills Co. Inc., Yokohama-shi, Kanagawa 230-0053 (JP); HARA, Y., c/o Ajinomoto Co. Inc., Kawasaki-shi, Kanagawa 210-8681 (JP); YAMADA, K., c/o Ajinomoto Co. Inc., Kawasaki-sh, Kanagawa 210-8681 (JP); SATO, H., c/oAjinomoto Co. Inc., Kawasaki-shi, Kanagawa 210-8681 (JP)
(74) Representative: Strehl Schübel-Hopf & Partner
(86) International application number: PCT/JP2002/004108
(87) International publication number: WO 2002/088286

(57) **Abstract**

It is intended to provide a sterol composition and a fat/oil composition, which have a higher effect of lowering the cholesterol level than commercially available phytosterol containing β-Sitosterol as a main component, and to provide foods containing the above compositions.

Thus, the present invention relates to a sterol composition having a total sterol content of 70% by weight or more and the ratio of Δ7-type sterol content of 13% by weight or more of the total sterol content; a fat/oil composition having a total sterol content of 1.0% by weight or more, preferably of 1.5% by weight or more, and the ratio of Δ7-type sterol content of 13% by weight or more, preferably of 25% by weight or more of the total sterol content; an agent for lowering cholesterol in serum, comprising the fat/oil composition; and food containing the fat/oil composition.

## Description

### Technical Field

The present invention relates to a sterol composition, fat/oil composition and foods, comprising a lot of sterol components that are superior in the effect of lowering cholesterol through their consumption in the same way as usual fat/oil comp ositions in a daily life.

### Background Art

Phytosterol is one of the effective base materials for lowering cholesterol (level) in serum. It is contained a lot in the seeds of plants, and also in edible, vegetable oil and fat in an amount of 0.1∼1.5% by weight.

The phytosterol is usually produced with extraction and purification of the distillate obtained in a deodorizing step during a purification process of vegetable oil and fat. It is well known that representative components of the phytosterol include β-sitosterol, campesterol and stigmasterol.

Many reports have been published concerning a cholesterol-lowering effect of the phytosterol, and its essential mechanism is considered to relate to an effect of inhibiting the absorption of cholesterol. Many attempts therefore have been made to increase an amount of phytosterol added in foods so that said inhibiting effect will be improved.

However, since solubility of the phytosterol in oil and fat is such low as 1∼2% by weight, problems have been recognized that addition of the phytosterol in an larger amount than the above level would deteriorate stability and properties of food goods, and that crystallized sterol in an insoluble state would be functionally inferior in solubility that is related to the absorption of cholesterol into a bowel micelle in a body.

Considering the mechanism that the phytosterol competes with cholesterol so as to inhibit the absorption of cholesterol in a small intestine, sterol ester needs to be hydrolyzed into free sterol (a free component) and fatty acid in order to sufficiently exert its biological effectiveness (Mattson, FH, et al., J. Nutr, 107:1139-1146 (1997)). However, sterol ester cannot be completely or 100 % hydrolyzed into free sterol (free component) and fatty acid since this reaction is reversible.

Accordingly, the free component of the sterol is superior to sterol ester itself in the effect of inhibiting the cholesterol absorption. Since a hydrolytic enzyme acts on the same site of sterol ester irrespective of the position of a double bond in the sterol, it is reasonable in terms of technical common knowledge to think that the above superiority of the free component to sterol ester should be commonly observed in any kind of the sterols. One of the examples in human, which shows the superiority of the free component to sterol ester in the effect of inhibiting the cholesterol absorption, is described in Mattson, FH, et al., The American Journal of Clinical Nutrition 35 April, 697-700 (1982).

On the other hand, as the solubility of the free component into oil and fat is low, its final utility will be reduced.

Some methods or technical means for overcoming the above problems have been proposed, such as (1) an invention to increase the solubility into oil and fat by reforming and processing the phytosterol, which is described in WO92-19640 (Raison Marganiinjoy) and Japanese Patent Laid-Open Application Hei. -237031(Unilever), and (2) an invention to increase the solubility or dispersibility by being emulsified, which is described in Japanese Patent Laid-Open Application Hei.11-146757(Unilever) and Japanese Patent Laid-Open Application 2000-102361(Mcneil-PPC, Incorporated), and (3) an invention to increase additive and synergistic effects, which is described in Japanese Patent Laid-Open Application hei.10-179086(Riken Vitamin Co., Ltd.) and Japanese Patent Laid-Open Application 2000-300191 (Nisshin Seiyu Co.).

However, the invention (1) has still disadvantages that the cost will be increased due to the necessity of reforming and processing, and that addition of the thus reformed sterol will have to be further increased when sterol ester is used in view of its functional inferiority in the effect of inhibiting the cholesterol absorption. Also the inventions (2) and (3) have disadvantages that the emulsion will become instable when contained in fat/oil such as frying oil that is used under heating or in O/W-type emulsion such as mayonnaise and source, which will restrict their utility.

Furthermore, since the sterol resources and materials used in the above techniques are based on edible oil seeds such as soybean and rape seeds, and phytosterol obtained as a by-product of pulp processing, no examination or study has been made about sterol components other than the phytosterol such as β-sitosterol, campesterol and stigmasterol.

The purpose of this invention is therefore to provide a sterol composition, fat/oil composition and foods, which comprise the sterol components that are superior in the effect of lowering cholesterol to commercially available vegetable sterols containing β-sitosterol as a main component.

The purpose of the present invention is also to provide a fat/oil composition and foods containing thereof having advantageous function and utility, which can solve the above disadvantages or problems such as precipitation of the crystalline due to the use of a large amount of phytosterol, especially its free component; instability of the emulsified composition; high cost and complexity in the reforming and processing.

The present inventors have devoted themselves to overcome the above problems, and finally found that Δ7-type sterols with a double bond at the 7-position in a sterol structure have a high effect of lowering cholesterol, and that the higher the ratio of Δ7 type sterol content in the total sterol is, the more the effect of lowering cholesterol in serum/liver will increase. They have also found that the effect of lowering cholesterol of Δ7-type sterols is attributed to their higher solubility into fat/oil than the commercially available phytosterol crystalline. These findings have finally led to the completion of the present invention.

### Disclosure of the Invention

The present invention relates to a sterol composition having a total sterol content of 70% by weight or more, preferably of 80% by weight or more, and the ratio of a Δ7-type sterol content of 13% by weight or more, preferably of 25% by weight or more of the total sterol content.

Sterol may include both its free component and ester in the sterol composition according to the present invention. However, in view of the effect of lowering cholesterol, the ratio of free components in the total sterol may amount preferably to 75% by weight or more, more preferably to 90% by weight or more, further more preferably to 95% by weight or more, and most preferably to 99% by weight or more in the sterol composition according to the present invention.

Further, the present invention is related to a fat/oil composition having a total sterol content of 1.0% by weight or more, preferably of 1.5% by weight or more, and the ratio of a Δ7 type sterol content of 13% by weight or more, preferably of 25% by weight or more of the total sterol content. Like the sterol composition of the present invention, the ratio of free sterol components in the total sterol may amount preferably to 75% by weight or more, more preferably to 90% by weight or more, further more preferably to 95% by weight or more, and most preferably to 99% by weight or more in the fat/oil composition according to the present invention. The present invention, however, excludes a fat/oil composition prepared from a soybean material with extraction without any addition of sterol.

As shown by the examples in the present specification, the fat/oil composition containing the sterol composition according to the present invention has a remarkable effect of lowering cholesterol in serum and/or liver. The present invention is therefore related also to an agent for lowering cholesterol, especially in serum. The agent according to the present invention may contain a total Δ7-type sterol amount of 0.1% by weight or more, preferably of 0.15% by weight or more. The agent for lowering cholesterol may also optionally contain any other components known in the art such as various auxiliaries and carriers.

The present invention is further related to cooking oil made of the above fat/oil composition, and a food containing the above fat/oil composition, especially those containing the total Δ7-type sterol amount of 0.1% by weight or more, preferably of 0.15% by weight or more.

### Best Mode for Carrying Out the Invention

Most of the phytosterol are generally a compound containing 27-29 carbon atoms, a hydroxyl group at 3-position, a branched chain with 8-11 carbon atoms at 17-position, and 1-3 double bonds.

The "total sterol" in the present specification means phytosterol components generally observed in oil seeds, and is specifically defined as a total amount of the following sterol components: Brasssicasterol(Δ5), Campesterol(Δ5), Stigmasterol(Δ5), β-Sitosterol(Δ5), Avenasterol(Δ5), Δ7-Avenasterol(Δ7), Spinasterol(Δ7), Δ7-Stigmastenol(Δ7), Gramisterol(Δ7), and Citrostadienol (Δ7).

The "Δ7-type sterol" in the in the present specification is defined as a sterol having a double bond at its 7-position, which includes Δ7-avenasterol(Δ7), spinasterol(Δ7), Δ7-stigmastenol(Δ7), gramisterol(Δ7), and citrostadienol (Δ7).

These Δ7-type sterols are hardly contained in commercially available phytosterol products that are prepared from the distillate of purified fat/oil based on soybean oil.

Cholesterol, on the other hand, is classified into Δ5-type sterol. β-sitosterol, campesterol and stigmasterol, which are contained in many oil seeds and well known as phytosterols to inhibit the cholesterol absorption, also belong to Δ5-type sterol.

The quantity of the Δ7-type sterols is determined in accordance with Standard Methods for the Analysis of Fats, Oils and Related Materials: Japan Oil Chemists' Society (1996) or an official method using GLC such as that defined by IUPAC (International Union of Pure and Applied Chemistry). Data for identifying each component such as their RRT (Relative Retention Time) are described in, for example, J. Jpn. Oil Chem Soc (YUKAGAKU), Vol.33, No.8, p46-50, 1984.

The ratio of the free components in the total sterol is analyzed in accordance with a method described in J. Agric. Food Chem, 44:2149 (1996).

### Specifically, it is carried out in the following conditions:

Column: LiChromosorbu Diol 5µm (4.0φ x 125mm) Merck Co.;
Column Temp.:40°C;
Detector: ELSD (Evaporative Light Scattering Detector;
Flowing rate: 1.0 ml/min;
Solvent: (A)hexane/acetic add=1000/1(v/v), (B)2-propanol;
Elution program: 0-8 min.(constant as A:B=100:0), 8-10 min.(linear gradient from A:B=100:0 to A:B=99:1), 10-30 min,( constant as A:B=99:1), 30-31 min.( linear gradient from A:B=99:1 to A:B=100:0).
Internal Standard: Cholesterol (Sigma: purity of 98% or more) for the free components, and cholesterol linolate (purity of 98% or more) for the esters;
Ratio of the free components in the total sterols:(total amount of the peaks of the free components)/(total amount of the peaks of the total sterols).

The sterol composition and fat/oil composition having a high ratio of the Δ7-type sterols according to the present invention may be prepared according to any method known to those skill in the art. For example, it may be prepared using as a starting material the oil seeds containing a lot of the Δ7-type sterols such as soybean, rape, palm, safflower, rice, sesame, sunflower, camellia, cucumbers, tea, and wheat germ; and their tissues such as germ and hull or fractions containing a lot of those sterols.

The composition according to the present invention may alternatively be prepared with the use of deodorized distillate containing a lot of sterols as starting resource, which has been produced in a purifying step, especially in a deodorizing step of the fat/oil obtained from the above oil seeds.

Furthermore, the sterol composition of the present invention may be prepared by any method generally known in the art. For example, a starting material made of the above distillate is mixed with alkali such as sodium hydroxide or potassium hydroxide, and saponificated. The resulting non-saponificated compounds are in turn extracted with ether or n-hexane, evaporated and dried. The resulting compounds are then dissolved in methanol or ethanol and cooled for crystallization, followed by separation and purification to give desired phytosterol products.

Alternatively, the Δ7-type sterols may be prepared by conversion from triterpenes, saturated sterols and Δ5-type sterols to the Δ7-type sterols, or by separation or concentration of the Δ7-type sterols by means of a separation membrane and the like.

When a fat/oil composition, which has been prepared from the oil seeds or their fractionated tissues with the use of a conventional extraction and purification of sterols and fat/oil, has predetermined ranges of the sterol content and the Δ7-type sterol ratio, such fat/oil composition may be directly used as the fat/oil composition or the food composition according to the present invention.

On the other hand, in case that the sterol content and the Δ7-type sterol ratio of the resulting fat/oil composition do not meet the requirements, or that the deodorized distillate is used as the starting resource, they will be subjected to a conventional concentration and separation to give the composition having the desired ranges of sterol content and the Δ7-type sterol ratio.

The fat/oil composition of the present invention may be further prepared by mixing a sterol composition having a high Δ7-type sterol ratio such as that according to the present invention with another fat/oil composition. For example, the fat/oil composition is heated to 80-100°C, mixed with the sterol composition having a lot of the Δ7-type sterols, dissolved, and cooled to a room temperature to give the fat/oil composition of the present invention. The fat/oil components contained in the above sterol composition and the fat/oil composition may be derived from the same or different plant(s).

The fat/oil used in the present invention includes commonly used edible oils such as liquid oil or solid fat of soybean oil, rape oil, cotton oil, corn oil, safflower oil, sunflower oil, sesame oil, olive oil, palm oil, palm kernel oil, coconut oil, cacao butter, lard, beef tallow, fish oil, perilla oil, flax seed oil, tung oil, and castor oil; and blending oil thereof; processed fat/oils such as hydrogenated oil, fractionated oil, and interesterificated oil; hydrolyzed fatty acid and its ester.

The cooking oil made of the above fat/oil composition according to the present invention includes those used for fried foods, roasted foods, grilled foods, salad, dipping oil, and seasonings, being specifically, for example, a salad oil, tempura oil(Japanese deep frying oil), and a shortening oil.

The food containing the fat/oil composition according to the present invention includes margarine, spread, mayonnaise, dressing, bakery mix, drink, desserts, ice cream, snack foods, processed meat, bread, cake, cookies, source, frozen foods, or chilled foods.

The amount of the fat/oil composition contained in the foods may vary depending on a kind of the foods and the like, the total Δ7-type sterol amount of preferably 0.1% by weight or more, more preferably 0.15% by weight or more being added for exerting sufficiently the useful effects of the Δ7-type sterols.

### Examples

The present invention will be explained in more detail with reference to the following examples, which should not be construed as limiting a technical scope of the present invention.

The analysis of each fat/oil component was carried out in accordance with Standard Methods for the Analysis of Fats, Oils and Related Materials: Japan Oil Chemists' Society.

### Example 1

### (Preparation of a sterol composition derived from soybean germ oil)

Selected whole soybeans were preliminarily heated for 30 min at 80°C, and crudely crushed by being put through a rubber roller while their hulls were peeled away. After a fraction containing the germ as a main component was concentrated and separated by sorting with air. The resulting germ-enriched fraction had a germ concentration of about 70 % by weight and the other parts consisted mainly of cotyledon.

The above soybean material (150kg) having the germ concentration of about 70% by weight was heated to 60°C and pressed into flakes by means of a flaking-roller. The flakes were treated with n-hexane to give crude oil. The resulting crude oil was subjected to a purification process generally used in the art, i.e., steps for degumming, alkali refining, bleaching and deodorizing to give soybean germ oil (15kg). Although the "germ" should be academically called a "hypocotyle", the term "germ" is used in the present specification in the same meaning as the hypocotyle.

The resulting soybean germ oil (10kg) was then subjected to saponification, and to liquid-liquid extraction with ethyl ether and water to give unsaponificable matters extracted in ethyl ether (330g), which was then evaporated, dried, mixed with ethanol and cooled to be crystallized to give a sterol composition according to the present invention (purity:85.4% by weight; Δ7-type sterol content:25.8% by weight; free components ratio:99% by weight). The analytical data of the sterol composition are shown in Table 1.

**Table 1**

| | Content (mg/ml) | Ratio (%) |
|---|---|---|
| Campesterol | 6429 | 7.5 |
| Stigmasterol | 5303 | 6.2 |
| β-Sitosterol | 51610 | 60.4 |
| Δ7-Stigmastenol | 6675 | 7.8 |
| Δ7-Avenasterol | 4636 | 5.4 |
| Citrostadienol | 10762 | 12.6 |
| Totol | 85415 | 100.0 |

### Example 2

### (Comparison of solubility of the sterol composition derived from soybean germ oil into fat/oil)

The sterol composition prepared in Example 1 was mixed with soybean oil (2.0% by weight in terms of sterols) and thoroughly stirred. The resulting mixture was kept at 37 ± 2°C for 2, 5 and 7 hours while being sometimes stirred. After the heating, it was filtered in a thermostatic chamber controlled at 37±2°C to remove undissolved crystalline. The sterol concentration of the resulting filtrate was determined by the method mentioned above. A commercially available phytosterol (Ezai Co., PhytosterolF (purity:89.6% by weight, ; Δ7-type sterol content:0.0% by weight; free components ratio:97% by weight) was used as a reference. The composition ratio of the reference is shown in Table 2.

**Table 2**

| | Content (mg/ml) | Ratio (%) |
|---|---|---|
| Brassicasterol | 6619 | 7.6 |
| Campesterol | 22244 | 25.7 |
| Stigmasterol | 18429 | 21.3 |
| β-Sitosterol | 39327 | 45.4 |
| Δ7-Stigmastenol | 0.0 | 0.0 |
| Δ7-Avenasterol | 0.0 | 0.0 |
| Citrostadienol | 0.0 | 0.0 |
| Total | 86619 | 100.0 |

As seen from the results summarized in Table 3 below, the solubility of the present sterol composition derived from soybean germ oil into lipid was higher than that of the commercial phytosterol of the reference.

**Table 3**

| | Sterol concentration in filtrate (mg/100g oil) | | |
|---|---|---|---|
| | 2 hr. | 5 hr. | 7hr. |
| Reference | 1210 | 1490 | 1540 |
| The sterol composition derived from soybean germ oil | 1840 | 1860 | 1900 |

### Example 3

### (Preparation of a sterol composition derived from sunflower oil)

In the same way as in Example 1, deodorized distillate of sunflower oil (10kg) was saponificated, and to liquid-liquid extraction with ethyl ether and water to give unsaponificable matters extracted in ethyl ether (330g), which was then evaporated, dried, mixed with ethanol and cooled to be crystallized to give a sterol composition according to the present invention (purity:73.1% by weight; Δ7-type sterol content:13.5% by weight; free components ratio:99% by weight). The analytical data of the sterol composition are shown in Table 4.

**Table 4**

| | Content (mg/ml) | Ratio (%) |
|---|---|---|
| Campesterol | 7755 | 10.6 |
| Stigmasterol | 7526 | 10.3 |
| β-Sitosterol | 47917 | 65.6 |
| Δ7-Stigmastenol | 4414 | 6.0 |
| Δ7-Avenasterol | 1616 | 2.2 |
| Citorostadienol | 3830 | 5.2 |
| Total | 73059 | 100.0 |

### Example 4

### (Animal Test Resume)

The same amount of the sterol composition prepared in Example 3 as a test oil and the commercially available phytosterol (Ezai Co., Phytosterol F) (reference) was added to diet, and animal tests were performed according to the following animal test protocols in order to compare their effects for lowering cholesterol level in serum, liver and feces.

### (Protocols)

SD Wistar rats (190 - 200g) were housed (10 rats per cage) for two weeks, freely fed with the diet.

### (Test Diet)

The test diet was prepared by mixing casein (20.0% by weight), cellulose (4.0% by weight), mineral mixture (4.0% by weight), vitamin mixture (1.0% by weight), cholesterol (0.5% by weight), sodium cholate (0.25% by weight), choline Cl (0.20% by weight), the sterol composition prepared in Example 3 or Phytosterol F (0.50% by weight in terms of sterols), soybean oil (10.0% by weight in terms of neutral fats except phytosterol contained therein) and sucrose making up the rest of 100%.

### (Analysis)

At the end of the test period, the rats were anesthetized with ethyl ether. Blood was collected from their abdominal aorta and livers were excised. Serum was separated from the blood by centrifugation for 15 min. at 3,000 rpm. The total cholesterol level in the serum was measured by an enzymatic analysis. The lipid was extracted from the liver and the cholesterol level was enzymatically determined in accordance with the method of Folch, J. LeeM. & Sloane-Stanley, GHA 1957 J. Biol. Chem., 226:497-509. The feces were collected for three days before autopsy, lyophilized and pulverized. The pulverized feces plus 5α-cholestane as an internal standard were saponificated with alkaline aqueous solution, extracted with n-hexane, and subjected to gas chromatography (TMS) for quantification.

### (Animal Test Results)

As seen from the results in Table 5 below, there observed a tendency that the cholesterol level in the serum was lower in Test Sample than in Reference Sample. Furthermore, the cholesterol level in the liver was significantly lower in Test Sample than in Reference Sample, showing that a less amount of cholesterol was accumulated in liver in the case of Test Sample. In contrast, the cholesterol level in the feces was higher in Test Sample than in Reference Sample, showing that cholesterol was defecated much more in Test Sample. These results revealed that the effect of lowering the cholesterol level of the Δ7-type sterols is higher than that of the usual phytosterols.

**Table 5**

| | Diet | Sterol Content (%diet) | Total cholesterol in serum (mg/dl) | Cholesterol in liver (mg/g liver) | Cholesterol in feces (mg/g feces) |
|---|---|---|---|---|---|
| Test | Soybean oil + the sterol composition of the present inv ention | 0.50 | 95 ± 28 | 32 ± 5 * | 40 ± 2 |
| Ref. | Soybean oil + The commercially available phytosterol | 0.50 | 100 ± 26 | 39 ± 6 | 35 ± 3 |

| | | | | | |
|---|---|---|---|---|---|
| Significant difference (*p <0.05) | | | | | |

### Example 5

The same animal tests as in Example 4 were performed, except that the sterol composition derived from soybean germ oil (the present invention prepared in Example 1) was used at an amount of 0.15% by weight and 0.50% by weight instead of the sunflower oil-derived sterol composition, and that the rats were housed for four weeks. The results obtained were shown in Table 6 below.

**Table 6**

| | Diet | Sterol Content (%diet) | Total cholesterol in serum (mg/dl) | Cholesterol in liver (mg/g liver) | Cholesterol in feces (mg/g feces) |
|---|---|---|---|---|---|
| Test No. 1 | Soybean oil + the sterol composition of the present inv ention | 0.15 | 98 ± 16* | 71 ± 7 | 35 ± 3 |
| Test No.2 | Soybean oil + the sterol composition of the present inv ention | 0.50 | 80 ± 14* | 52 ± 8 * | 44 ± 2** |
| Ref. No.1 | Soybean oil + The commercially available phytosterol | 0.15 | 116 ± 20 | 71 ± 7 | 32 ± 3 |
| Ref. No.2 | Soybean oil + The commercially available phytosterol | 0.50 | 101 ± 36 | 65 ± 8 | 37 ± 3 |

| | | | | | |
|---|---|---|---|---|---|
| Significant differences (*p <0.05, **p <0.01) | | | | | |

### (Animal Test Results)

As seen from the results in Table 6, the cholesterol levels in the serum were significantly lowered in Test Samples No. 1 and No.2 when compared with Reference Samples No.3 and No.4 having the same sterol content, respectively. Furthermore, while the cholesterol level in the liver was significantly lower in Test Sample No.2 than in Reference Sample No.4, the cholesterol level in the feces was significantly higher in Test Sample No.2 than in Reference Sample No.4. These results showed again that the effect of lowering the cholesterol level of the Δ7-type sterols is higher than that of the usual phytosterols. Furthermore, they demonstrate the effectiveness not only of the fat/oil composition of the present invention but also of the foods of the present invention containing the Δ7-type sterols according to the present invention.

### Example 6

### (Animal Test Resume)

The same amount of the sterol composition prepared in Example 1 as a test oil and the commercially available phytosterol (Ezai Co., Phytosterol F) (Reference) was added to diet. However, sodium cholate that is an absorption-promoting agent was not added in the diet. Since the cholesterol level in serum almost never changed in this system, it would be easier to access an input and output of cholesterol in a body by determining the levels of cholesterol in liver and feces (including coprostanol) according to the following animal test protocols. Since cholesterol will be converted into coprostanol by enterobacteria, coprostanol is considered as defecated cholesterol.

### (Protocols)

SD Wistar rats (190 ∼ 200g) were housed (8 rats per cage) for four weeks, freely fed with the diet.

### (Test Diet)

The test diet was prepared by mixing casein (20.0% by weight), cellulose (4.0% by weight), mineral mixture (4.0% by weight), vitamin mixture (1.0% by weight), cholesterol (0.5% by weight), choline Cl (0.20% by weight), the phytosterol composition prepared from soybean germ oil (0.35% by weight in terms of sterols), soybean oil (10.0% by weight in terms of neutral fats except phytosterol contained therein) and sucrose making up the rest of 100%.

### (Analysis)

At the end of the test period, the rats were anesthetized with ethyl ether. Blood was collected from their abdominal aorta and livers were excised. The lipid was extracted from the liver and the cholesterol level was enzymatically determined in accordance with the method of Folch, J. LeeM. & Sloane-Stanly, GHA 1957 J. Biol. Chem., 226:497-509. The feces were collected for three days before autopsy, lyophilized and pulverized. The pulverized feces plus 5α-cholestane as an internal standard were saponificated with alkaline aqueous solution, extracted with n-hexane, and subjected to gas chromatography (TMS) for quantification.

### (Animal Test Results)

**Table 7**

| | Diet | Sterol Content (%diet) | Cholesterol in liver (mg/g liver) | Cholesterol in feces (mg/g feces) |
|---|---|---|---|---|
| Test No.1 | Soybean oil + the sterol composition of the present invention | 0.35 | 225 ± 15 | 53 ± 1 |
| Ref. No.1 | Soybean oil + The commercially available phytosterol | 0.35 | 272 ± 17 | 48 ± 3* |

| | | | | |
|---|---|---|---|---|
| Significant difference (*p <0.05) Cholesterol in feces (Cholesterol + Coprostanol) | | | | |

As seen from the results in Table 7, while the cholesterol level in the liver was significantly lower in Test Sample No.1 than in Reference Sample No.1, the cholesterol level in the feces was significantly higher in Test Sample No.1 than in Reference Sample No.1. These results showed that the effect of inhibiting absorption of cholesterol of the Δ7-type sterols is higher than that of the usual phytosterols. Furthermore, they demonstrate the effectiveness not only of the fat/oil composition of the present invention but also of the foods of the present invention containing the Δ7-type sterols according to the present invention.

### Industrial applicability

It is confirmed that the sterol composition and the fat/oil composition of the present invention, which are characterized by their Δ7-type sterol content, have the effect of lowering the cholesterol level. The foods containing the fat/oil composition may be taken like usual fat/oil or their composition in a daily life, and a smaller amount of them may efficiently reduce the cholesterol level in serum and/or liver. As a result, they contribute a lot to the solution of the problems in the production and stability of foods, and to the reduction of the cost and complexity in the reforming and processing, making it possible to apply the above compositions in a variety of fields.

The sterol composition and the fat/oil composition according to the present invention also have a great advantage that a smaller amount of the same compositions may exert the same cholesterol-lowering effect in serum as the usual phytosterols do. They are also of a great value in application since they can significantly improve the problems in quality and cost, which will be encountered with the use of a large amount of the conventional phytosterols.

## Claims

1. A sterol composition having a total sterol content of 70% by weight or more and the ratio of a Δ7-type sterol content of 13% by weight or more of the total sterol content.

2. A sterol composition having a total sterol content of 80% by weight or more and the ratio of a Δ7-type sterol content of 25% by weight or more of the total sterol content.

3. A sterol composition according to Claim 1 or 2, wherein the ratio of free components in the total sterol amounts to 90% by weight or more.

4. A fat/oil composition having the total sterol content of 1.0% by weight or more, and the ratio of a Δ7-type sterol content of 13% by weight or more of the total sterol content, except that prepared from a soybean material with extraction without any addition of sterol.

5. A fat/oil composition according to Claim 4, having the total sterol content of 1.5% by weight or more.

6. A fat/oil composition according to Claim 4 or 5, having the ratio of Δ7 type sterol content of 25% by weight or more of the total sterol content.

7. A fat/oil composition according to any one of Claim 4∼6, wherein the ratio of free components in the total sterol amounts to 90% by weight or more

8. An agent for lowering cholesterol comprising the sterol composition according to any one of Claim 1 to 3.

9. An agent for lowering cholesterol in serum, comprising the fat/oil composition according to any one of Claim 4 to 7.

10. Cooking oil made of the fat/oil composition according to any one of Claim 4∼7.

11. A food containing the fat/oil composition according to any one of Claim 4∼7.

12. A food containing a total Δ7-type sterol amount of 0.1% by weight or more.

13. A food containing a total Δ7-type sterol amount of 0.15% by weight or more.

14. A food according to any one of Claim 11 to 13, which is selected from margarine, spread, mayonnaise, dressing, bakery mix, drink, desserts, ice cream, snack foods, processed meat, bread, cake, cookies, source, frozen foods, or chilled foods.
